(19) Europäisches Patentamt
European Patent Office
Office européen des brevets

(11) Numéro de publication: **0 267 127 B1**

(12) **FASCICULE DE BREVET EUROPEEN**

(45) Date de publication du fascicule du brevet: **06.12.89**

(51) Int. Cl.⁴: **C07C 69/63,** C07C 67/08

(21) Numéro de dépôt: **87420265.8**

(22) Date de dépôt: **30.09.87**

(54) **Procédé de préparation de trifluoroacétate d'éthyle.**

(30) Priorité: **16.10.86 FR 8614606**

(43) Date de publication de la demande: **11.05.88 Bulletin 88/19**

(45) Mention de la délivrance du brevet: **06.12.89 Bulletin 89/49**

(84) Etats contractants désignés: **AT BE CH DE ES FR GB GR IT LI LU NL SE**

(56) Documents cités: **Néant**

(73) Titulaire: **RHONE-POULENC CHIMIE, 25, quai Paul Doumer, F-92408 Courbevoie Cédex(FR)**

(72) Inventeur: **Amiet,Louis, 10 Quai Saint-Vincent, 69001 Lyon(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

EP 0 267 127 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de préparation de trifluoroacétate d'éthyle. Elle concerne plus particulièrement un procédé de préparation de trifluoroacétate d'éthyle présentant une grande pureté.

La préparation de trifluoroacétate d'éthyle est décrite dans la publication Research Disclosure n ° 244 032. Il est connu d'après cette publication de préparer les esters de l'acide trifluoroacétique par un procédé en 4 étapes selon lequel :

- on condense l'alcool de formule ROH, dans laquelle R représente un radical alkyle contenant 1 à 5 atomes de carbone, avec l'acide trifluoroacétique en présence d'un acide minéral fort (HC1, $H_2$ $SO_4$ ou $H_3$ $PO_4$) à une température de 25 à 110°C ;
- on refroidit le mélange obtenu et on élimine la phase aqueuse ;
- on traite la phase organique avec de l'acide phosphorique et/ou de l'acide sulfurique concentré, puis on poursuit la réaction entre l'acide et l'alcool à 25 - 110°C ;
- on isole l'ester par distillation.

Ce procédé ne permet pas, lorsque l'on part d'acide trifluoroacétique et d'éthanol, d'obtenir l'ester pur à plus de 99 % de manière économiquement rentable.

En effet la réaction d'estérification est une réaction équilibrée selon le schéma réactionnel suivant :

$$CF_3\ COOH + CH_3\ CH_2\ OH \rightleftharpoons CF_3\ COO\ CH_2\ CH_3 + H_2O$$

Pour permettre de déplacer l'équilibre on peut mettre un excès de l'un ou de l'autre des produits de départ.

Si on met un excès d'éthanol on ne peut plus le séparer totalement du trifluoroacétate d'éthyle obtenu car il forme avec ce dernier un azéotrope ayant un point d'ébullition proche de celui de trifluoroacétate d'éthyle. Si l'on met un excès d'acide trifluoroacétique, l'excès se dissout en partie dans la phase aqueuse contenant l'acide sulfurique d'où il est pratiquement impossible de l'extraire quand l'acide sulfurique est utilisé en faible quantité. L'acide trifluoroacétique étant une matière première onéreuse, on cherche à réduire sa perte au minimum.

Aussi l'industrie recherche depuis longtemps un procédé de préparation de trifluoroacétate d'éthyle pur et économiquement rentable.

La présente invention a permis d'atteindre cet objectif. Elle a pour objet un procédé de préparation de trifluoroacétate d'éthyle caractérisé en ce que :

- dans une première étape on met en contact l'acide trifluoroacétique avec un léger excès d'éthanol en présence d'acide sulfurique concentré et d'un solvant du trifluoroacétate d'éthyle présentant un point d'ébullition supérieur à celui de l'acide trifluoroacétique, une densité inférieure à 1 et ne formant pas d'azéotrope avec le trifluoroacétate d'éthyle puis on procède à une décantation avec élimination de la coche sulfurique suivie d'une éventuelle étape supplémentaire où l'on ajoute de l'acide sulfurique concentré pour compléter l'estérification ;
- dans une deuxième étape on introduit une quantité supplémentaire d'acide sulfurique et d'acide trifluoroacétique puis on procède à une décantation en éliminant la couche sulfurique ;
- dans une troisième étape on effectue une distillation du mélange obtenu à la deuxième étape avec récupération du trifluoroacétate d'éthyle pur.

Ce procédé est une amélioration nette du procédé décrit dans le document publié dans la revue Research Disclosure n ° 244 032. En effet, il permet d'obtenir un ester présentant une pureté supérieure à 99 %.

Afin de déplacer l'équilibre réactionnel au cours de cette première étape l'éthanol sera introduit en excès par rapport à la stoechiométrie d'au maximum 10 % et de préférence d'environ 5 %.

La réaction est effectuée en présence d'acide sulfurique qui joue le rôle d'agent déshydratant du milieu réactionnel et de catalyseur.

Pour jouer son rôle déshydratant l'acide sulfurique utilisé est de préférence l'acide concentré du commerce. On en utilise de préférence au moins 3 % en poids par rapport à l'acide trifluoroacétique mis en oeuvre. Une quantité d'acide sulfurique calculée en poids par rapport à l'acide trifluoroacétique comprise entre 5,5 et 10 % est encore plus préférentiellement utilisée. Une quantité supérieure n'est pas nuisible au procédé de l'invention mais n'apporte aucun avantage supplémentaire. La quantité d'acide sulfurique sera aisément adaptée par l'homme de l'art à l'économie du procédé.

Le solvant ajouté au milieu réactionnel a pour fonction de favoriser la démixtion du milieu en allégeant la phase organique par rapport à la phase minérale constituée d'acide sulfurique et d'eau. Il présente ainsi de préférence un densité inférieure à 1. Il doit permettre la solubilisaton de l'ester et de l'excès d'acide trifluoroacétique. Afin de faicliter ensuite la séparation de l'ester et du solvant, ce dernier de préférence ne doit pas former d'azéotrope avec l'ester et doit présenter de préférence un point d'ébullition supérieur à celui de l'ester et de l'acide trifluoroacétique.

Parmi les solvants utilisables dans le procédé de l'invention on peut citer de façon non limitative le toluène et les xylènes. Etant utilisé comme solvant il sera introduit en quantité pondérale au moins égale à deux fois celle de l'acide trifluoroacétique et de préférence d'environ 300 g par mole d'acide trifluoroacétique.

Lors de la démixtion du mélange obtenu après réaction de l'acide sur l'alcool, on ne perd pas d'acide trifluoroacétique dans la phase aqueuse la présence du solvant favorise son passage dans la phase organique. L'alcool utilisé en excès reste en majorité dans la phase aqueuse et est éliminé.

L'éthanol étant une matière première beaucoup moins onéreuse que l'acide trifluoroacétique il est préférable de perdre celui-ci.

La phase organique contenant, elle, un léger excès d'éthanol est mise en contact dans une deuxième étape avec une quantité supplémentaire d'acide trifluoroacétique et d'acide sulfurique. La quantité d'acide trifluoroacétique introduite doit permettre d'estérifier tout l'alcool subsistant dans le milieu. On ajoute ainsi de préférence au moins 10 % en poids d'acide trifluoroacétique par rapport au poids d'acide trifluoroacétique introduit lors de la première étape et de préférence une quantité comprise entre 10 et 20 %, encore plus préférentiellement cette quantité sera comprise 15 et 20 %.

La quantité d'acide sulfurique concentré introduite lors de la deuxième étape est d'au-moins 5 % en poids par rapport au poids d'acide trifluoroacétique et d'éthanol introduit lors de la première étape et de préférence d'environ 7 %.

Après réaction finale de l'alcool et de l'acide, le mélange décante et se sépare en deux phases, la phase inférieure constituée de l'acide sulfurique et de l'eau provoquée par l'estérification peut éventuellement être recyclée vers la première étape du procédé selon l'invention.

La phase organique constituée de l'ester, de l'excès d'acide trifluoroacétique et du solvant subit une distillation au cours de laquelle on sépare :

- une fraction de tête contenant un mélange dont la point d'ébullition est compris entre 54 et 55°C constituée d'eau et d'ester qui décante facilement. L'ester est alors recyclé vers la deuxième étape du procédé, l'eau est éliminée.
- une deuxième fraction constituée de trifluoroacétate d'éthyle présentant une pureté supérieure à 99 % dont le point d'ébulliton est de 60°C.
- une troisième fraction constituée essentiellement d'un azéotrope trifluoroacétate d'éthyle-acide trifluoroacétique dont le point d'ébullition est de 73°C, d'un peu d'ester et d'un peu de solvant, qui est recyclée éventuellement à la dernière étape d'estérification
- le résidu de distillation constitué essentiellement de toluène peut être éventuellement réintroduit à l'étape 1.

L'invention sera plus complètement décrite à l'aide de l'exemple suivant.

On schématisera au cours des deux exemples suivants le terme acide trifluoroacétique par TFA, et trifluoroacétate d'éthyle par TFAE.

<u>1er exemple avec toluène comme solvant auxiliaire 3 étapes d'estérification (voir schéma 1)</u>

<u>Cycle I</u>

On utilise un réacteur de laboratoire de 2 litres, équipé de systèmes d'agitation, de chauffage, régulation, distillation (colonne de laboratoire de 30 plateaux réels - environ 22 plateaux théoriques, type "Oldershaw"), condensation. Le réacteur est muni d'un système permettant la décantation et le soutirage des liquides.

. <u>1ère étape (a)</u> :

On introduit dans l'ordre, dans le réacteur, 3 moles de TFA (1), puis, sous légère agitation en 0,5 heure environ, 3,15 moles d'éthanol (2) ce qui provoque une hausse de température de 25°C environ. On ajoute alors 30 g de $H_2SO_4$ (3) à 98 % et 12 g d'eau pour se rapprocher plus vite et simuler le système à l'équilibre, lorsqu'on introduira au lieu de $H_2SO_4$ pur la phase sulfurique déjà hydratée provenant du 2ème stade. On porte à reflux 1 heure, puis arrête le chauffage et introduit 900 g de toluène (4) pur. On agite pendant environ 10 mn puis stoppe et laisse décanter. Les 2 couches liquides se séparent presqu'immédiatement et complètement (pas de trouble).

La couche inférieure, d'un poids de 84 g, est soutirée.

. <u>1ère étape (b)</u> :

On ajoute au réacteur 30 g de $H_2SO_4$ (3) pur à 98 %, on chauffe à reflux 1 h 30 mn sous agitation, puis refroidit à 20°C, laisse décanter et soutire la couche inférieure pesant 43 g, qui est conservée ($A_b$).

. <u>2ème étape</u> :

On rajoute 30 g de $H_2SO_4$ (3) à 98 % et 0,5 mole de TFA (1). On chauffe à reflux sous agitation 1 h 30 mn. La température atteint 59°C en tête de colonne et 86°C dans le bouilleur. On décante, après refroidissement à 70°C, une couche inférieure pesant 30 g qui est conservée (A).

. <u>3ème étape : distillation</u> :

Avec un taux de reflux d'environ 4, on passe entre 58 et 60°C une 1ère fraction de 26 g relarguant 1 g d'eau. Par prélèvement l'analyse indique 1,5 % d'éthanol, 2 % de TFA et 36 % de TFAE. Après prélèvement, on recycle les 18 g (B) restant au bouilleur.

On distille ensuite à température fixe de 60°C une fraction de 288 g à 99,5 % de TFAE, constituant la production d'ester,

Puis on sépare de 60 à 110°C, avec un palier à 73°C, une fraction pesant 196 g, contenant 0,53 mole de TFA, qui est mise en réserve (C).

On laisse ensuite refroidir.

<u>Cycle II</u>

Au liquide (toluène essentiellement) restant dans le réacteur :

<u>1ère étape (a)</u> :

On ajoute 3 moles de TFA, 3,15 moles d'éthanol et la fraction ($A_b$). Après chauffage au reflux 1 heure et refroidissement, on sépare une couche inférieure de 94 g qui est éliminée.

. <u>1ère étape (b)</u> :

On rajoute A, porte à reflux 1 h 30 mn sous agitation et en opérant comme au 1er stade, sépare la couche inférieure pesant 33 g, mise en réserve (A'b).

. <u>2ème étape</u> :

On ajoute 30 g de $H_2SO_4$ pur à 98 % et la fraction (C). On porte à reflux sous agitation 1 h 30 mn.

On opère comme précédemment et recueille une couche inférieure de 31 g (A').

. <u>3ème étape : distillation</u> :

-On sépare une fraction passant à 59 - 60°C en tête de colonne, se séparent en 7 g d'eau et 13 g d'une couche organique (B') dont l'analyse indique qu'elle est constituée à 97 % de TFAE, 0,28 % de TFA, 0,99 % d'éthanol et 0,48 % de toluène.

-On sépare ensuite à 60°C une fraction de 417 g contenant 99,7 % de TFAE, 0,1 % d'éthanol, 0,04 % de toluène et 0,15 % de TFA.

-On sépare de 60 à 110°C, avec un palier à 73°C, une fraction de 172 g contenant 0,49 mole de TFA (C').

Puis on stoppe la distillation et laisse refroidir.

<u>Cycle III</u>

. <u>1ère étape (a)</u> :

On opère comme au cycle précédent en ajoutant au liquide restant dans le réacteur 3 moles de TFA, 3,15 moles d'éthanol et (A'b). La couche aqueuse acide éliminée pèse 79 g.

. <u>1ère étape (b)</u> :

On ajoute (A') et opère comme précédemment. On sépare une couche aqueuse acide de 38 g (A"b).

. 2ème étape :

On ajoute cette fois la fraction C′ et 30 g de $H_2SO_4$ neuf. On sépare la couche acide pesant 31 g (A″).

. 3ème étape : distillation :

On sépare une 1ère fraction de 57 à 60°C formée de 5 g d'eau et 28 g de produit à 98;6 % d'ester (B″), puis la fraction constituant la production d'ester passant à température fixe de 60°C, pesant 417 g et contenant d'après l'analyse 99,7 % d'ester, 0,09 % d'éthanol, 0,03 % de toluène et 0,06 % de TFA. La dernière fraction (C″) passant de 60 à 110°C pèse 157 g et contient : 43 % de TFA, 54 % de TFAE et environ 3 % de toluène ; elle est entièrement utilisable pour un nouveau cycle, ainsi que la 1ère fraction de 28 g (B″).

Le résidu de distillation pèse 850 g.

2ème exemple avec toluène 2 étapes d'estérification (voir schéma 2)

Dans chaque cycle d'estérification on fait réagir les mêmes quantités de TFA et d'éthanol que dans l'exemple précédent, et de la même façon. On enchaîne la réalisation de cet exemple avec le précédent en utilisant certaines fractions recyclables et la même charge de solvant restant de la distillation de la 3ème étape de l'exemple précédent.

Cycle I

. 1ère étape :

Au résidu de distillation du dernier cycle de l'exemple précédent on ajoute 3 moles de TFA (1), 3,15 moles d'éthanol (2), et (A″). Après le processus habituel on sépare une fraction aqueuse acide de 70 g contenant 0,47 % de F total et moins de 1 mg/kg d'ion $F^-$.

. 2ème étape :

On introduit 30 g de $H_2SO_4$ (3) neuf et (C″) qui représente 0,59 mole de TFA puis sépare une couche inférieure de 46 g (A″ ′).

La distillation fournit une première fraction de 7 g d'eau et 18 g d'ester (B″ ′), passant de 58 à 60°C, puis, une deuxième fraction passant à 60°C de 411 g, enfin une fraction (C) de 61 à 110°C de 159 g contenant 0,37 mole de TFA.

Cycle II

. 1ère étape :

On opère comme précédemment en utilisant A″ ′ comme acide, on sépare une couche acide aqueuse de 90 g, contenant 0,22 % de F total et moins de 1 mg/kg d'ion $F^-$.

. 2ème étape :

On ajoute B″ ′, C″ ′ contenant un excès de TFA (0,37 mole) et 30 g de $H_2SO_4$ (3) neuf. On sépare une couche de 43 g (A″″).

. 3ème étape : distillation :

La 1ère fraction donne 4 g d'eau et 45 g (B″″) d'ester recyclable. La deuxième fraction, à 60°C, pèse 453 g. On sépare ensuite 91 g contenant 0,29 mole de TFA (C″″).

Cycle III

. 1ère étape :

On opère comme précédemment en utilisant (A″″) comme source de $H_2SO_4$. On sépare une couche aqueuse de 84 g.

. 2ème étape :

et C″″ et 0,20 mole de TFA (1) neuf, et 30 g de $H_2SO_4$ (3).

La couche acide aqueuse séparée pèse 36 g.

. <u>3ème étape : distillation</u> :

On sépare une 1ère fraction de 57 à 60°C, pesant 41 g et contenant 98,3 % de TFAE, une 2ème fraction de 426 g passant à 60°C, enfin une 3ème fraction passant de 60 à 110°C avec palier à 73°C, d'un poids de 118 g et contenant 28,2 % de TFA, 44,4 % de TFAE et 27,3 % de toluène.

<u>3ème exemple avec utilisation de xylène à la place de toluène selon le schéma 2</u>

On effectue 2 cycles dans l'appareillage de laboratoire, en remplaçant le toluène par du xylène technique.

<u>Cycle I</u>

. <u>1ère étape</u> :

Mise en réaction de 3 moles de TFA (1), 3,15 moles d'éthanol (2) en présence de 900 g de xylène (4) technique et de 30 g de $H_2SO_4$ (3) concentré. On chauffe le mélange à reflux et sous agitation pendant 1 h 30 mn. La température dans le bouilleur est de 78°C. Puis on refroidit à environ 40°C et décante. On sépare la couche inférieure pesant 78 g.

. <u>2ème étape</u> :

On ajoute au milieu réactionnel 30 g de $H_2SO_4$ (3) concentré et 0,5 mole de TFA (1) et chauffe à reflux sous agitation, pendant 1 h 30 mn, puis refroidit à environ 50°C, décante et sépare la couche inférieure pesant 36 g, soit A cette fraction.

. <u>3ème étape : distillation</u> :

On sépare une fraction de 53 à 60°C en tête, se séparant après condensation en une couche inférieure d'ester de 137 g (soit B) et une couche supérieure de 7 g, éliminée. On recueille ensuite à 60°C en palier une fraction de 286 g et 99,5 % de TFAE, puis, de 60 à 135°C, une fraction (soit C) de 91 g contenant d'après l'analyse, 0,42 mole de TFA. On stoppe l'opération et refroidit le réacteur bouilleur.

<u>Cycle II</u>

. <u>1ère étape</u> :

On introduit sur le liquide restant dans le réacteur à la fin du cycle précédent 3 moles de TFA (1), puis 3,15 moles d'éthanol (2), la fraction acide (A) pesant 36 g. On opère comme dans le cycle I et sépare une couche aquo-sulfurique de 91 g à 20°C.

. <u>2ème étape</u> :

On ajoute au contenu du réacteur les fractions (B) et (C) du cycle précédent, puis 30 g de $H_2SO_4$ (3) concentré et 0,08 mole de TFA (1). Au reflux, la température atteint 56°C en tête de colonne et 82°C dans le bouilleur. Après refroidissement à environ 40°C on sépare la couche inférieure décantée, pesant 33 g.

. <u>3ème étape : distillation</u> :

On distille de 55 à 60°C une fraction se séparant en 3 g d'eau (couche supérieure) et 141 g d'ester, à 99,4 % de TFAE ; puis à 60°C une fraction de 400 g à 99,7 % de TFAE ; enfin une fraction passant de 60 à 137°C pesant 103 g et contenant, d'après l'analyse, 0,44 mole de TFA.

## Revendications

1. Procédé de préparation de trifluoroacétate d'éthyle caractérisé en ce que :
- dans une première étape on met en contact l'acide trifluoroacétique avec un léger excès d'éthanol en présence d'acide sulfurique concentré et d'un solvant présentant un point d'ébullition supérieur à celui de l'acide trifluoroacétique, une densité inférieure à 1 et ne formant pas d'azéotrope avec le trifluoroacétate d'éthyle puis on procède à une décantation avec élimination de la couche sulfurique suivie d'une éventuelle étape supplémentaire où l'on ajoute de l'acide sulfurique concentré pour compléter l'estérification ;
- dans une deuxième étape on introduit une quantité supplémentaire d'acide sulfurique et d'acide trifluoroacétique puis on procède à une décantation en éliminant la couche sulfurique ;

- dans une troisième étape on effectue une distillation du mélange obtenu à la deuxième étape avec récupération du trifluoroacétate d'éthyle pur.

2. Procédé selon revendication 1, caractérisé en ce que l'excès d'éthanol est compris entre 2 et 10 % par rapport à la stoechiométrie et de préférence d'environ 5 %.

3. Procédé selon revendication 1, caractérisé en ce que la quantité d'acide sulfurique concentré introduite à la première étape est d'au-moins 3 % en poids par rapport à l'acide trifluoroacétique introduit.

4. Procédé selon revendicaton 1, caractérisé en ce que le solvant est choisi parmi le toluène et le xylène et est de préférence le toluène.

5. Procédé selon les revendication 1 et 4, caractérisé en ce que la quantité pondérale de toluène utilisée par rapport à la quantité totale d'acide trifluoracétique introduit est d'au moins 2 et de préférence d'environ 300 g par mole d'acide trifluoroacétique introduit.

6. Procédé selon revendication 1, caractérisé en ce que la quantité supplémentaire d'acide sulfurique introduite au cours de la deuxième étape est d'au moins 5 % en poids par rapport au poids d'acide trifluoroacétique et d'éthanol introduit lors de la première étape et de préférence d'environ 7 %.

7. Procédé selon revendication 1, caractérisé en ce que la quantité supplémentaire d'acide trifluoroacétique introduite au cours de la deuxième étape est d'environ 10 % à 20 % en poids par rapport au poids d'acide trifluoroacétique introduit lors de la première étape.

## Patentansprüche

1. Verfahren zur Herstellung von Ethyltrifluoracetat, dadurch gekennzeichnet daß:

– man in einem ersten Schritt Trifluoressigsäure mit einem leichten Überschuß an Ethanol in Gegenwart von konzentrierter Schwefelsäure und einem Lösungsmittel in Kontakt bringt, das einen Siedepunkt oberhalb dessen der Trifluoressigsäure hat, eine Dichte unterhalb von 1 hat und kein Azeotrop mit Ethyltrifluoracetat bildet, daß man dann eine Dekantierung mit Eliminierung des Schwefelsäureschicht durchführt, gefolgt von einem eventuellen zusätzlichen Schritt in dem man konzentrierte Schwefelsäure zugibt, um die Veresterung zu vervollständigen;

– man in einem zweiten Schritt eine zusätzliche Menge an Schwefelsäure und Trifluoressigsäure zugibt und dann eine Dekantierung durchführt, um die Schwerfelsäureschicht zu entfernen;

– man in einem dritten Schritt eine Destillation des Gemischs durchführt, das im zweiten Schritt erhalten wird, unter Gewinnung von reinem Ethyltrifluoracetat.

2. Verfahren nach Anspruch 1, dadurch gekennzeichnet, daß der Überschuß an Ethanol zwischen 2 und 10% bezogen auf die Stöchiometrie beträgt, vorzugsweise ungefähr 5%.

3. Verfahren nach Anspruch 1, dadurch gekennzeichnet, daß die Menge an konzentrierter Schwefelsäure, die im ersten Schritt zugegeben wird, wenigstens 3 Gew.% bezogen auf die zugegebene Menge an Trifluoressigsäure beträgt.

4. Verfahren nach Anspruch 1, dadurch gekennzeichet, daß das Lösungsmittel ausgewählt wird aus Toluol und Xylol und vorzugswiese Toluol ist.

5. Verfahren nach den Ansprüchen 1 und 4, dadurch gekennzeichnet, daß die Gewichtsmenge an Toluol die verwendet wird, bezogen auf die Gesamtmenge, an zugegebener Trifluoressigsäure wenigstens 2 und vorzugsweise ungefähr 300 g pro Mol zugegebener Trifluoressigsäure beträgt.

6. Verfahren nach Anspruch 1, dadurch gekennzeichnet, daß die zusätzliche Menge an Schwefelsäure, die im Verlauf des zweiten Schritts zugegeben wird, wenigstens 5 Gew.%, bezogen auf das Gewicht der im ersten Schritt zugegebenen Trifluoressigsäure und des Ethanols beträgt und vorzugsweise ungefähr 7%.

7. Verfahren nach Anspruch 1, dadurch gekennzeichnet, daß die zusätzliche Menge an Trifluoressigsäure, die im Verlauf des zweiten Schritts zugegeben wird, ungefähr 10% bis 20 Gew.%, bezogen auf das Gewicht der während des ersten Schritts zugegebenen Trifluoressigsäure, beträgt.

## Claims

1. Process for the preparation of ethyl trifluoroacetate, characterized in that:

in a first stage, trifluoroacetic acid is brought into contact with a slight excess of ethanol in the presence of concentrated sulphuric acid and of a solvent, having a boiling point higher than that of trifluoroacetic acid and a density less than 1 and which does not form an azeotrope with ethyl trifluoroacetate and a phase separation is then carried out, the sulphuric layer being removed, followed, if required, by an additional stage in which concentrated sulphuric acid is added in order to complete the esterification;

in a second stage, an additional quantity of sulphuric acid and trifluoroacetic acid is introduced and a phase separation is then carried out, the sulphuric layer being removed; and

in a third stage, the mixture obtained in the second stage is distilled, pure ethyl trifluoroacetate being recovered.

2. Process according to claim 1, characterized in that the excess of ethanol is between 2 and 10% relative to the stoichiometry and preferably approximately 5%.

3. Process according to claim 1, characterized in that the quantity of concentrated sulphuric acid introduced in the first stage is at least 3% by weight relative to the trifluoroacetic acid introduced.

4. Process according to claim 1, characterized in that the solvent is chosen from amongst toluene and xylene and is preferably toluene.

5. Process according to claims 1 and 4, characterized in that the quantity by weight of toluene employed relative to the total quantity of trifluoroacetic acid introduced is at least twice and preferably approximately 300g per mole of trifluoroacetic acid introduced.

6. Process according to claim 1, characterized in that the additional quantity of sulphuric acid introduced during the second stage is at least 5% by weight relative to the weight of trifluoroacetic acid and of ethanol introduced during the first stage and preferably approximately 7%.

7. Process according to claim 1, characterized in that the additional quantity of trifluoroacetic acid introduced during the second stage is approximately 10% to 20% by weight relative to the weight of trifluoroacetic acid introduced during the first stage.

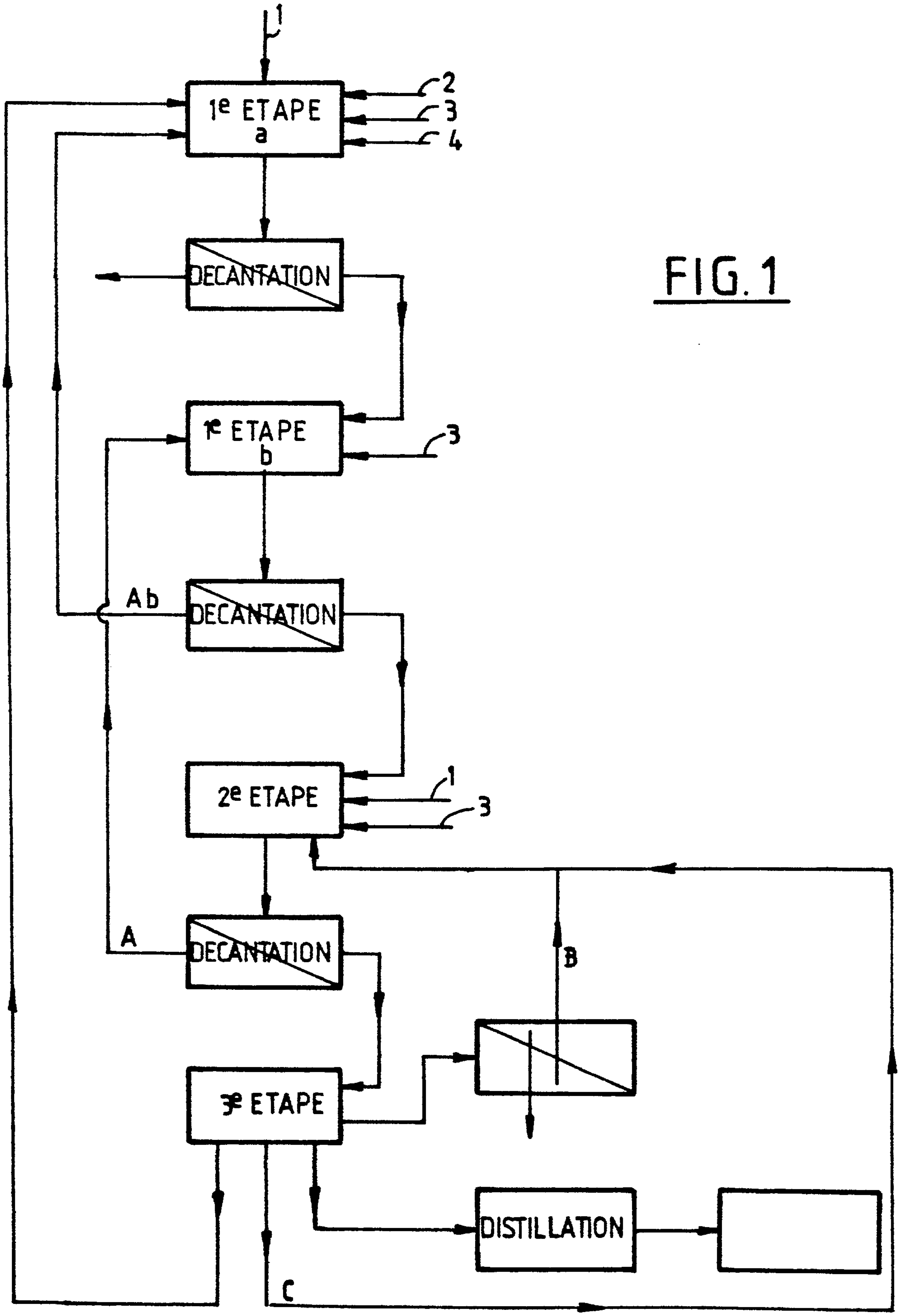
1
1e ETAPE a
2
3
4
DECANTATION
1e ETAPE b
3
Ab
DECANTATION
2e ETAPE
1
3
A
DECANTATION
B
3e ETAPE
DISTILLATION
C

FIG. 1

FIG. 2

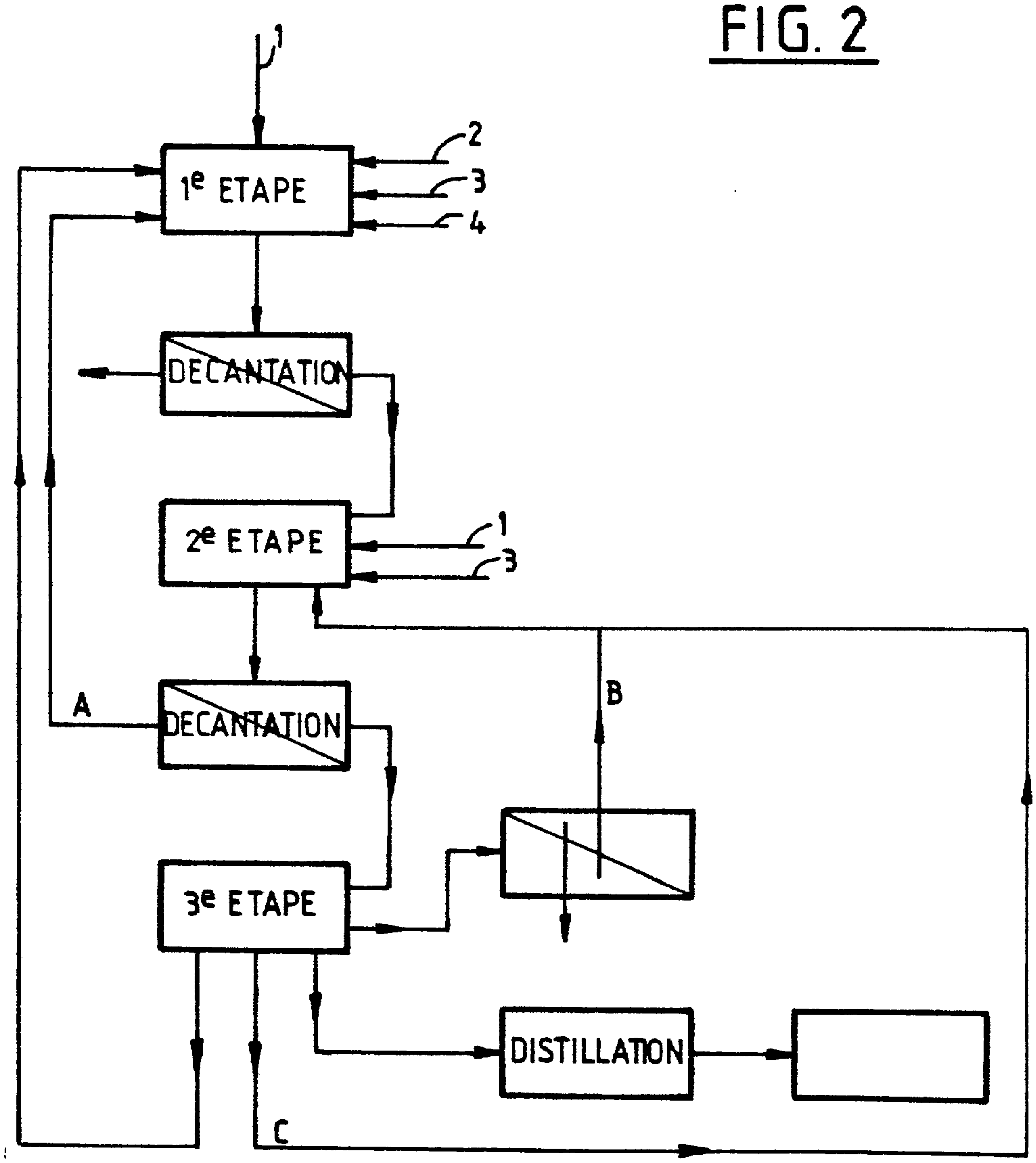
1
2
1e ETAPE
3
4
DECANTATION
2e ETAPE
1
3
B
A
DECANTATION
3e ETAPE
DISTILLATION
C